# EUROPEAN PATENT APPLICATION

(11) **EP 1 889 626 A1**
(43) Date of publication of application: **20.02.2008**
(21) Application number: 07075601.0
(22) Date of filing: 12.07.2007
(51) Int. Cl.: A61K 38/16, A61K 38/45, A61K 39/00, A61K 48/00, C12Q 1/48, C12Q 1/68

(54) **Hip kinase for fertility control**

(30) Priority: 16.08.2006 EP 06076585
(71) Applicant: Bayer Schering Pharma AG, 13353 Berlin (DE)
(72) Inventor: Sacher, Frank, 13353 Berlin (DE); Fritsch, Martin, 10555 Berlin (DE); Feldman, Richard I., El Cerrito California 94530 (US); Möller, Carsten, 10627 Berlin (DE)

(57) **Abstract**

The present invention relates to the use of a pharmaceutical composition which comprises as active component at least one HipK4 nucleic acid, a HipK4 polypeptide, modulators of HipK4, antibodies directed against HipK4, a HipK4 antisense, and a HipK4 RNAi, for the diagnosis and treatment of fertility impairments and for contraception. The invention further relates to the use of this pharmaceutical composition for manufacturing a medicament for the treatment of fertility impairments.

SEQUENCE LISTING

<110>  Schering AG

<120>  Hip kinase for fertility control

<130>  53441

<160>  4

<170>  PatentIn version 3.1

<210>  1
<211>  2451
<212>  DNA
<213>  homo sapiens

<400>  1
agagccactc aacagcgctg gaacccattc ggtggggcct ggggcccctc atcccaagcc      60
aggagggttt ctggggaggg gtgcagcccc tggcagactg acagtgtggc ctgggggttt     120
gggggtgcca gggaagcagg ggccaacctc ataggaggag acacgagtgc ggttctcttt     180
cccccactgg ggggcctgct gtgtcagcag ccaggcggga ggcctgggcg gcagagccag     240
tggtacaggg gcctgggcag ggcggtgtct ggcagcagcg gcaccatgtc caccatccag     300
tcggagactg actgctacga catcatcgag gtcttgggca aggggacctt cggggaggta     360
gccaagggct ggcggcggag cacgggcgag atggtggcca tcaagatcct caagaatgac     420
gcctaccgca accgcatcat caagaacgag ctgaagctgc tgcactgcat gcgaggccta     480
gaccctgaag aggcccacgt catccgcttc cttgagttct tccatgacgc cctcaagttc     540
tacctggtct ttgagctgct ggagcaaaac cttttcgagt tccagaagga gaacaacttc     600
gcgcccctcc ccgcccgcca catccgtaca gtcaccctgc aggtgctcac agccctggcc     660
cggctcaagg agctggctat catccacgct gatctcaagc ctgagaacat catgctggtg     720
gaccagaccc gctgcccctt cagggtcaag gtgattgact tcggatccgc cagcattttc     780
agcgaggtgc gctacgtgaa ggagccatac atccagtcgc gcttctaccg ggcccctgag     840
atcctgctgg ggctgccctt ctgcgagaag gtggacgtgt ggtccctggg ctgcgtcatg     900
gctgagctgc acctgggctg gcctctctac cccggcaaca acgagtacga ccaggtgcgc     960

tacatctgcg aaacccaggg cctgcccaag ccacacctgt tgcacgccgc ctgcaaggcc 1020

caccacttct tcaagcgcaa cccccaccct gacgctgcca acccctggca gctcaagtcc 1080

tcggctgact acctggccga gacgaaggtg cgcccattgg agcgccgcaa gtatatgctc 1140

aagtcgttgg accagattga gacagtgaat ggtggcagtg tggccagtcg gctaaccttc 1200

cctgaccggg aggcgctggc ggagcacgcc gacctcaaga gcatggtgga gctgatcaag 1260

cgcatgctga cctgggagtc acacgaacgc atcagcccca gtgctgccct gcgccacccc 1320

ttcgtgtcca tgcagcagct gcgcagtgcc cacgagacca cccactacta ccagctctcg 1380

ctgcgcagct accgcctctc gctgcaagtg gaggggaagc cccccacgcc cgtcgtggcc 1440

gcagaagatg ggacccccta ctactgtctg gctgaggaga aggaggctgc gggtatgggc 1500

agtgtggccg gcagcagccc cttcttccga gaggagaagg caccaggtat gcaaagagcc 1560

atcgaccagc tggatgacct gagtctgcag gaggctgggc atgggctgtg gggtgagacc 1620

tgcaccaatg cggtctccga catgatggtc cccctcaagg cagccatcac tggccaccat 1680

gtgcccgact cgggccctga gcccatcctg gccttctaca gcagccgcct ggcaggccgc 1740

cacaaggccc gcaagccacc tgcgggttcc aagtccgact ccaacttcag caacctcatt 1800

cggctgagcc aggtctcgcc tgaggatgac aggccctgcc ggggcagcag ctgggaggaa 1860

ggagagcatc tcggggcctc tgctgagcca ctggccatcc tgcagcgaga tgaggatggg 1920

cccaacattg acaacatgac catggaagct gagaggccag accctgagct cttcgacccc 1980

agcagctgtc ctggagaatg gctgagtgag ccagactgca ccctggagag cgtcaggggc 2040

ccacgggctc aggggctccc accccgccgc tcccaccagc atggtccacc ccggggggcc 2100

accagcttcc tccagcatgt caccgggcac cactgatggt gattccaccc ctgcccatca 2160

ctgggggctg cgctagctgg gctggcattc cctcccaacc tgaactgctc ctcagagcca 2220

tctcctgaac ccacaaatta ttcttacaga aagatagtta tccagaaatt ctcattcccc 2280

gtctgcggtg cggtgcgtgc ctgcacacct ctcctaaaca cagcagggct ttggagtctg 2340

gcccatgctc cttggccaga aggacagcag gaaagggggc tgcaccccgc tggccctgcg 2400

ctcgcccttg gccctgctgc ctctgtctat ttcaatatag aactgttcag c 2451

<210> 2

<211> 616

<212> PRT

<213> Homo sapiens

<400> 2

Met Ser Thr Ile Gln Ser Glu Thr Asp Cys Tyr Asp Ile Ile Glu Val
1               5                   10                  15

Leu Gly Lys Gly Thr Phe Gly Glu Val Ala Lys Gly Trp Arg Arg Ser
            20                  25                  30

Thr Gly Glu Met Val Ala Ile Lys Ile Leu Lys Asn Asp Ala Tyr Arg
        35                  40                  45

Asn Arg Ile Ile Lys Asn Glu Leu Lys Leu Leu His Cys Met Arg Gly
    50                  55                  60

Leu Asp Pro Glu Glu Ala His Val Ile Arg Phe Leu Glu Phe Phe His
65                  70                  75                  80

Asp Ala Leu Lys Phe Tyr Leu Val Phe Glu Leu Leu Glu Gln Asn Leu
                85                  90                  95

Phe Glu Phe Gln Lys Glu Asn Asn Phe Ala Pro Leu Pro Ala Arg His
            100                 105                 110

Ile Arg Thr Val Thr Leu Gln Val Leu Thr Ala Leu Ala Arg Leu Lys
        115                 120                 125

Glu Leu Ala Ile Ile His Ala Asp Leu Lys Pro Glu Asn Ile Met Leu
    130                 135                 140

Val Asp Gln Thr Arg Cys Pro Phe Arg Val Lys Val Ile Asp Phe Gly
145                 150                 155                 160

Ser Ala Ser Ile Phe Ser Glu Val Arg Tyr Val Lys Glu Pro Tyr Ile
                165                 170                 175

Gln Ser Arg Phe Tyr Arg Ala Pro Glu Ile Leu Leu Gly Leu Pro Phe
            180                 185                 190

Cys Glu Lys Val Asp Val Trp Ser Leu Gly Cys Val Met Ala Glu Leu
        195                 200                 205

His Leu Gly Trp Pro Leu Tyr Pro Gly Asn Asn Glu Tyr Asp Gln Val
    210                 215                 220

Arg Tyr Ile Cys Glu Thr Gln Gly Leu Pro Lys Pro His Leu Leu His
225                 230                 235                 240

Ala Ala Cys Lys Ala His His Phe Phe Lys Arg Asn Pro His Pro Asp
                245                 250                 255

Ala Ala Asn Pro Trp Gln Leu Lys Ser Ser Ala Asp Tyr Leu Ala Glu
            260                 265                 270

Thr Lys Val Arg Pro Leu Glu Arg Arg Lys Tyr Met Leu Lys Ser Leu
        275                 280                 285

Asp Gln Ile Glu Thr Val Asn Gly Gly Ser Val Ala Ser Arg Leu Thr
    290                 295                 300

Phe Pro Asp Arg Glu Ala Leu Ala Glu His Ala Asp Leu Lys Ser Met
305                 310                 315                 320

Val Glu Leu Ile Lys Arg Met Leu Thr Trp Glu Ser His Glu Arg Ile
                325                 330                 335

Ser Pro Ser Ala Ala Leu Arg His Pro Phe Val Ser Met Gln Gln Leu
            340                 345                 350

Arg Ser Ala His Glu Thr Thr His Tyr Tyr Gln Leu Ser Leu Arg Ser
        355                 360                 365

Tyr Arg Leu Ser Leu Gln Val Glu Gly Lys Pro Pro Thr Pro Val Val
    370                 375                 380

Ala Ala Glu Asp Gly Thr Pro Tyr Tyr Cys Leu Ala Glu Glu Lys Glu
385                 390                 395                 400

Ala Ala Gly Met Gly Ser Val Ala Gly Ser Ser Pro Phe Phe Arg Glu
                405                 410                 415

Glu Lys Ala Pro Gly Met Gln Arg Ala Ile Asp Gln Leu Asp Asp Leu
            420                 425                 430

Ser Leu Gln Glu Ala Gly His Gly Leu Trp Gly Glu Thr Cys Thr Asn
        435                 440                 445

Ala Val Ser Asp Met Met Val Pro Leu Lys Ala Ala Ile Thr Gly His
    450                 455                 460

His Val Pro Asp Ser Gly Pro Glu Pro Ile Leu Ala Phe Tyr Ser Ser
465                 470                 475                 480

Arg Leu Ala Gly Arg His Lys Ala Arg Lys Pro Pro Ala Gly Ser Lys
                485                 490                 495

Ser Asp Ser Asn Phe Ser Asn Leu Ile Arg Leu Ser Gln Val Ser Pro
            500                 505                 510

Glu Asp Asp Arg Pro Cys Arg Gly Ser Ser Trp Glu Glu Gly Glu His
        515                 520                 525

Leu Gly Ala Ser Ala Glu Pro Leu Ala Ile Leu Gln Arg Asp Glu Asp
    530                 535                 540

Gly Pro Asn Ile Asp Asn Met Thr Met Glu Ala Glu Arg Pro Asp Pro
545                 550                 555                 560

Glu Leu Phe Asp Pro Ser Ser Cys Pro Gly Glu Trp Leu Ser Glu Pro
                565                 570                 575

Asp Cys Thr Leu Glu Ser Val Arg Gly Pro Arg Ala Gln Gly Leu Pro
            580                 585                 590

Pro Arg Arg Ser His Gln His Gly Pro Pro Arg Gly Ala Thr Ser Phe
        595                 600                 605

Leu Gln His Val Thr Gly His His
    610                 615

## Description

The present invention relates to the enzyme Hip kinase 4 which is closely associated with male infertility. It is accordingly suitable for the diagnosis and treatment of male infertility and as means for creating contraceptives.

Currently, about 15% of all couples in Germany are unintentionally childless, and the proportion thereof is continuously increasing. The reasons for the infertility derive equally from the man and the woman. Whereas the reasons in women have been substantially researched and can be diagnosed, in men it is possible to establish organic causes in only about 30% of cases. About one third of the remaining cases can be attributed to oligospermia of unknown origin, whereas the reasons for the remaining cases are still unclear at the present state of knowledge. In particular, impairment of the sperm maturation which takes place in the epididymis is suspected in cases of idiopathic infertility where, despite an adequate number of sperm in the ejaculate, no fertilization of the female ovum takes place (H.W.G. Baker, Male Reproductive Dysfunction, 341 ff, 1986).

A large number of enzymatically controlled processes are intermeshed in sperm maturation. It is to be assumed that impairments caused by dysregulation of an enzyme lead to impairments of sperm maturation and thus to sperm incapable of fertilization.

The object of the present invention is to provide novel means with which impairments of enzymic metabolism in the human testes, and especially male infertility, can be diagnosed and treated, and which can be employed for fertility control.

Hip kinase 4 belongs to a new family of enzymes which interact in a yeast two-hybrid screen with domains of other proteins. These proteins comprise a conserved protein kinase domain and a domain which interacts with other proteins, and therefore the protein kinases are referred to as homeodomain-interacting protein kinases (HipKs). To date, 4 isoforms are known: HipK1, HipK2, HipK3 and HipK4 (Kim Choi et al. 1998, J Biol Chem, 273(40): 25875-9). These protein kinases belong to a larger family of kinases which are regulated by tyrosine phosphorylation and catalyse their autophosphorylation on serine/threonine residues and on tyrosine residues, but phosphorylate their substrates only on serine/threonine residues (Zhang, Li et al. 2005, Genomics 85(1): 117-30) . The HipK1, 2 and 3 have been detectable in the cell nucleus, and it has likewise been possible to identify some substrates.

Less is known to date about HipK4. The sequence of HipK4 was claimed for the first time in the application WO 04/087901 (Wyeth).

However, no functional information has to date been available for HipK4 (Kim, Choi et al. 1998, J Biol Chem 273(40): 25875-9; Moilanen, Karvonen et al. 1998, Mol Biol Cell 9(9): 2527-43; Rochat-Steiner, Becker et al. 2000, J Exp Med 192(8): 1165-74; Ecsedy, Michaelson et al. 2003, Mol Cell Biol 23(3): 950-60; Zhang, Li et al. 2005; Zhang, Nottke et al. 2005, Proc Natl Acad Sci USA 102(8): 2802-7).

It has now been possible to show for the first time in the present invention that HipK4 plays an important role in the processes of spermatogenesis/spermiogenesis.

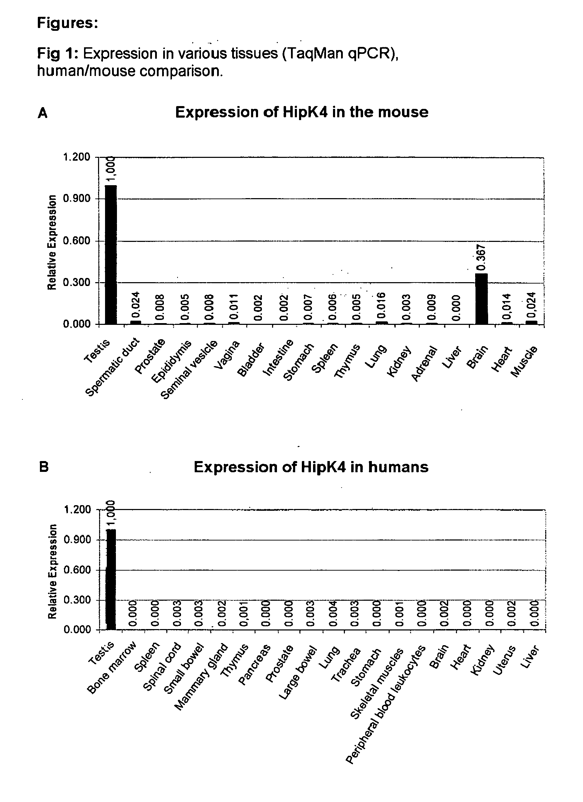

It has been possible to show that HipK4 is expressed in particular in the testes. The quantitative tissue distribution of Hip kinase 4 was determined in mice and humans by means of Q-RT-PCR (Fig. 1 A and B). HipK4 is expressed in particular in the testes in mice and in humans. Expression was additionally detectable in the mouse brain, showing 37% of the intensity of the level of expression in the testis (Fig. 1 B).

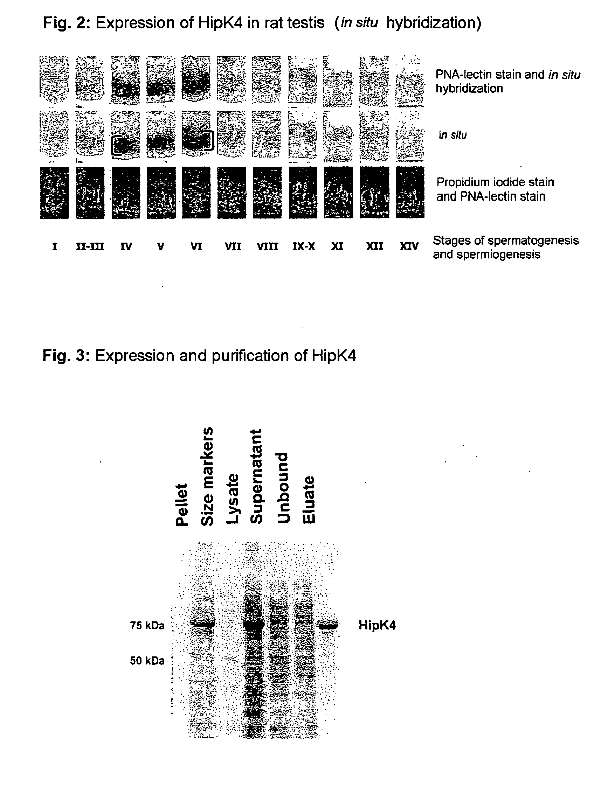

Detailed investigation of the expression of HipK4 by means of in situ hybridization in the rat testis was able to demonstrate that HipK4 is expressed exclusively in post-meiotic round spermatids (Fig. 2). In combination with a PNA-lectin (actrosome staining) and propidium iodide staining it was possible to restrict expression of HipK4 during spermiogenesis to development stage 4-6 (Fig. 2). This virtually testis-specific HipK4 expression, which is confined to post-meiotic round spermatids, strengthens the essential role of Hip kinase 4 in post-meiotic spermiogenesis and thus supports the possibility of developing a means for diagnosing fertility impairments and for contraception based on inhibition of HipK4.

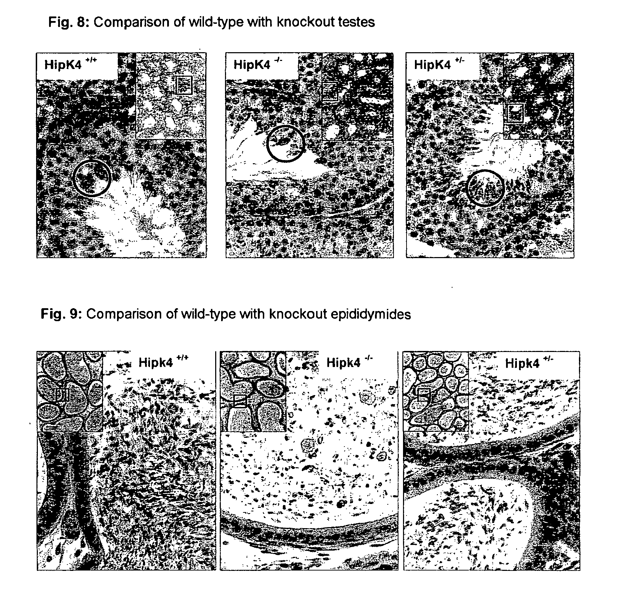

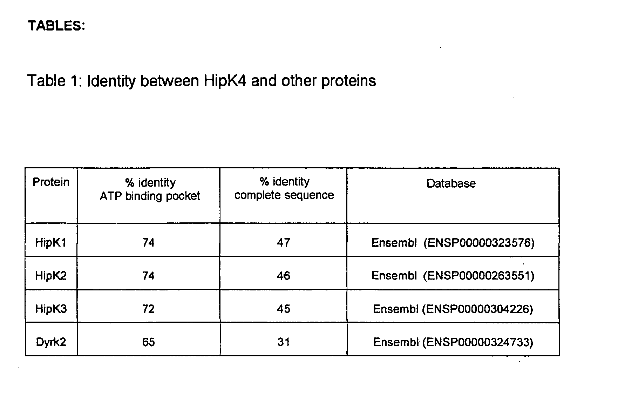

Using a HipK4-knockout mouse line it was possible by histological analysis of testis and epididymis to show that HipK4 plays an essential role during spermiogenesis (Fig. 8-9). Deficiency of HipK4 in the testis leads to an influence on spermiogenesis, as shown by an untypical arrangement of the developing spermatids in the seminiferous tubules (Fig. 8). A morphological change in spermatids is to be observed most greatly in the stages shortly before the release of the sperm into the lumen of the seminiferous tubules (Fig. 8 circle). Moreover, the sperm of the HipK4-deficient mouse line no longer shows an elongate shape which is characteristic of this stage of development, but show an altered morphology. Eventually, this defective spermiogenesis leads to release of a drastically reduced number of sperm in the testis and thus to an accumulation of these deformed sperm in the tail of epididymis (Fig. 9).

Analysis of the HipK4-deficient mouse line was able unambiguously to demonstrate the essential function of this kinase in spermiogenesis. It is thus to be assumed that the absence of HipK4 leads to infertility. Consequently, HipK4 modulators can be developed as novel medicament for contraception, and analysis of HipK4 functionality can be utilized to diagnose infertility.

The present invention therefore solves the present problem by providing a novel therapeutic method in which the enzyme Hip kinase 4 and modulators of this enzyme serve as target substance for developing novel medicaments which are suitable both for the diagnosis of infertility and for fertility control.

The present invention relates to the use of a pharmaceutical composition which contains as active component at least one substance selected from the group of a HipK4 nucleic acid corresponding to Seq ID No. 1, a HipK4 polypeptide corresponding to Seq ID No. 2, modulators of HipK4, antibodies directed against HipK4, a HipK4 antisense, and a HipK4 RNAi, for the diagnosis and treatment of fertility impairments, for contraception and also for manufacturing a medicament for the treatment of fertility impairments.

HipK4 nucleic acids include both single- or doublestranded DNA, e.g. cDNA and RNA, e.g. mRNA, cRNA, pre-mRNA, and parts thereof. The DNA and protein sequences are depicted in Seq ID No. 1 and 4 (Seq ID No. 1: HipK4 human nucleotide sequence ID ENST00000291823 and Seq ID No. 2: HipK4 human protein sequence ID ENSP00000291823). The nucleic acid depicted in Seq ID No. 1 codes for a human HipK4 protein.

It is preferred to use a pharmaceutical composition in which the nucleic acid includes a protein-coding segment of the nucleic acid sequence depicted in Seq ID No. 1. A protein-coding segment of the sequence depicted in Seq ID No. 1 is located in the 286-2136 region.

The nucleic acid can be obtained from mammals, e.g. human cells, or from a cDNA library or a genomic library which is obtained for example from human cells. It can be isolated by known techniques using short segments of the nucleic acid sequence shown in Seq ID No. 1 as hybridization probes or amplification primers.

The invention further relates to the abovementioned use of a pharmaceutical composition in which the polypeptide includes the amino acid sequence depicted in Seq ID No. 2.

The polypeptide may be a recombinant polypeptide, a natural, isolated polypeptide or a synthetic polypeptide.

The mRNA of the polypeptide according to Seq ID No. 2 is transcribed in testes and in germ cells.

The present invention relates to the abovementioned use of a pharmaceutical composition which comprises a polypeptide fragment corresponding to the amino acid sequence shown in Seq ID No. 2 from position 1 to 616.

The HipK4 polypeptide or partial regions thereof (peptides) can be used to produce antibodies. To produce polyclonal antibodies, the polypeptides or peptides can be bound for example to KLH (keyhole limpet haemocyanin) and injected into animals, e.g. rabbits. They can also be used to produce monoclonal antibodies. A HipK4 polypeptide or peptide or a mixture of a plurality of HipK4 peptides can be used for producing antibodies. The antibodies are produced in this connection by standard methods as described for example in Kohler, G. and Milstein, C., Nature 1975, 256, 495-497 and Nelson, P.N. et al., Mol. Pathol. 2000, 53, 111-117.

The invention relates to the use of a pharmaceutical composition which comprises antibodies which are directed against the HipK4 polypeptide for diagnosing fertility impairments.

The antibodies can be used to detect the HipK4 polypeptides. This is possible for example by immunohistochemistry. The antibodies can also be employed in other immunoassays such as, for example, an ELISA (enzyme linked immunosorbent assay) or in radioimmunoassays. Thus, the concentration of polypeptide in tissue or cell extracts can be detected.

Expression of the polypeptide can also be detected by detecting mRNA in the cells. The invention therefore also relates to the use of a probe with nucleic acid sequences which are complementary to the nucleic acid sequences which codes for the polypeptide which includes the amino acid sequence depicted in Seq ID No. 2 for preparing a reagent for detecting the presence of mRNA in cells. A probe is a short piece of RNA or DNA having at least 14 nucleotides. The probes of the invention can be used for example in a Northern blot analysis. This method is described for example in Sambrook, J. et al., 1989, Cold Spring Harbor Laboratory Press. Other methods for detecting RNA are in situ hybridization, RNAse protection assay or PCR.

The invention additionally relates to the use of a pharmaceutical composition which comprises molecules which are directed against the nucleic acid sequence and are able to suppress expression of the HipK4 protein, such as, for example, antisense molecules and synthetic molecules which inhibit the stability or translation of HipK4 mRNA. Molecules of these types can be employed specifically for contraception.

The present invention also relates to the use of a composition which comprises as active component at least one HipK4 nucleic acid, or a HipK4 polypeptide or a HipK4 antisense molecule as target substance for producing a composition for the treatment of diseases which are causally related to the HipK4 gene and/or protein.

The invention further relates to methods for diagnosing disorders whose causes include mutations of the HipK4 protein. It is possible to use DNA chips for this purpose. The invention therefore relates to a DNA chip on which is immobilized at least one oligonucleotide which corresponds to the complete cDNA sequence or a partial sequence or complementary sequence to that described in Seq ID No. 1. The invention thus further relates to the use of a DNA chip of the invention for diagnosing fertility impairments inter alia in the testis and oviduct.

DNA chips, also known as DNA microarrays, are miniaturized supports, usually made of glass or silicon, on whose surface DNA molecules of known sequence are immobilized in high density in an ordered array. The surface-bound DNA molecules are hybridized with complementary, possibly labelled nucleic acids. The label may be a fluorescent dye.

In the case of oligonucleotide chips, the oligonucleotides which can be bound to a DNA chip of the invention represent partial sequences of the gene products (mRNA, or cDNA derived therefrom). One or more oligonucleotides can be bound per gene on the DNA chip. Oligonucleotides with a length of 25 nucleotides are preferred. These are preferably selected from the respective 3' untranslated end of the gene. Methods for producing and using DNA chips are described for example in US patents Nos. 5,578,832; 5,556,752 and 5,510,270.

In the case of cDNA chips, the complete gene products (cDNAs) or subfragments (200-500 bp long) are bound on the chip. The method is described for example in Eckmann L et al., J. Biol. Chem., 2000, 275(19), 14084-14094.

Firstly, the suitable DNA sequences as shown in Seq ID No. 1 are determined. Suitable sequences are those able to hybridize with the selected gene transcripts. The oligonucleotides are then prepared on the chip by a chemical process based on photolithographic methods. The photolithographic masks used for this purpose are generated by suitable computer algorithms.

The labelled RNA is incubated with the chip in a hybridization oven. Subsequently, the chip is analysed in a scanner which determines the hybridization profiles. It is thus possible to establish whether there are changes in the transcript (e.g. mutations, truncations). It likewise makes it possible to quantify the transcript and thus the HipK4 protein and makes it possible to draw conclusions about for example a mutation in the promoter.

The invention further relates to methods based on the use of RNAi (RNA interference) for modifying the HipK4 transcript. Such an application can be used specifically for creating contraceptives. The oligonucleotides employed in the RNAi are based on Seq ID No. 1 and lead to modification of the transcript through interaction with the endogenous HipK4 RNA.

The invention additionally relates to the use of a pharmaceutical composition which comprises as active component a HipK4 nucleic acid which can be used in the form of a gene therapy approach for the treatment of male infertility. Gene therapy means all methods which treat the cause of genetically related disorders by modifying the genome. In this method, the missing genetic information is supplied to the body directly. Vectors comprising the gene are injected or inhaled and thus reach their site of action.

The invention further relates to cells which are transfected with a nucleic acid sequence coding for the HipK4 receptor, or a vector. Examples of cells which can be used are E. coli, yeast, Pichia, Sf9, HEK-293, CHO, COS, CV-1 or BHK. These cells can be used to produce the HipK4 polypeptide or for assay systems.

The present invention also relates to a method for preparing the HipK4 polypeptides, characterized in that the host cells are cultured under conditions which permit expression of the DNA sequence, and whereby the expression product can be obtained from the culture mixture.

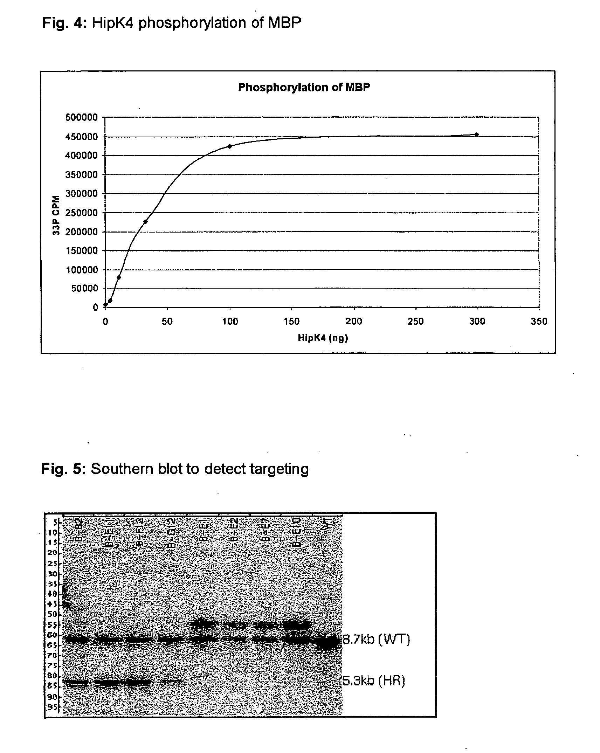

The function of a protein kinase consists of phosphorylating specific substrate proteins. This generally leads to a modification of the biochemical property of the substrate, possibly being manifested by the change in the state of activity or the binding properties. Development of a means for contraception can now be based on regulating the activity of a kinase. However, the precondition for this is that the protein to be regulated displays an enzymatic activity and acts not just as binding partner within intracellular communication. Demonstration that HipK4 possesses intrinsic kinase activity takes place by a kinase assay in which the general kinase substrate MBP is employed (Fig. 4). The intensity of the phosphorylation takes place as a function of the amount of HipK4 employed. MBP with its property as a general kinase substrate comprises a large number of serine and threonine phosphorylation residues and is thus not an ideal substrate for identifying inhibitors and investigating kinase kinetics. An ideal substrate has been identified by a systematic investigation of a peptide library, the sequence of the peptides employed being based on known phosphorylation sites and activation loops of described substrates.

The proteins identified as HipK4 substrates with the aid of such an experimental approach are the following, inter alia; FoxO3a (NP_963853) and RBL2 (NM_005611). It is of interest that FoxO3a and RBL2 demonstrably have an important function in spermatogenesis or are essential for fertility (Yan, Kero et al. 2001; Hosaka, Biggs et al. 2004). The fact that not only HipK4 but also the substrates phosphorylated by HipK4 are important constituents of the control of human and mouse fertility suggest the essential function of the kinase activity of HipK4 in the relevant signalling pathways. However, identification of HipK4 substrates may not only contribute to elucidating the biological function, but also generate information necessary for further analysis and development of HipK4 modulators. In this connection, the peptides identified as substrate are employed for establishing an HTS technology optimized for HipK4.

It was possible to show by sequence comparisons that HipK4 shares only 74% sequence identity in the ATP binding pocket with its closest homologues, HipK1 and HipK2 (Table 1). Such a low homology with other proteins, specifically in the ATP binding pocket which represents the principal target region of putative inhibitors, facilitates the development of a specific HipK4 modulator, because it can be assumed that the HipK4 modulators will, because of the small sequence identity, not bind further proteins.

The present invention likewise relates to the use
a) of a HipK4 nucleic acid,
b) of a HipK4 polypeptide, or
c) of a cell which expresses HipK4
for identifying effectors of a HipK4 polypeptide.

Effectors are substances which have inhibitory or activating activity on the HipK4 polypeptide and are able to influence the HipK4 function of the HipK4 polypeptides.

The invention further relates to an assay system for identifying effectors of a HipK4 polypeptide, where a HipK4 polypeptide can be incubated as a whole or partial sequences thereof with a modulator, and for example the binding of a molecule to the HipK4 polypeptide is measured. The invention further relates to an assay system for identifying effectors of the HipK4 polypeptide. This entails purified enzyme being incubated with radiolabelled ATP and the substrate MBP and, after an appropriate incubation time, the degree of phosphorylation of the substrate being determined (Fig. 4).

The present invention likewise relates to a method for providing a pharmaceutical composition, where
a) substances are contacted with an assay system for identifying HipK4 effectors,
b) the effect of the substances on the assay system is measured by comparison with controls,
c) a substance which shows a modulation of the activity of the HipK4 polypeptide in step b) is identified,
d) and the substance identified in step c) is mixed with formulating materials customary in pharmacy.

The effectors of the HipK4 polypeptide can be employed both for the diagnosis, for promoting fertility and for contraception in men.

The preferred preparations consist of a dosage form which is suitable for oral, enteral or parenteral administration. Examples of such dosage forms are tablets, film-coated tablets, sugar-coated tablets, pills, capsules, powders or depot forms, and suppositories. Appropriate tablets can be obtained for example by mixing the active ingredient with known excipients, for example inert diluents such as dextrose, sugar, sorbitol, mannitol, polyvinylpyrrolidone, disintegrants such as corn starch or alginic acid, binders such as starch or gelatin, lubricants such as carboxypolymethylene, carboxymethylcellulose, cellulose acetate phthalate or polyvinyl acetate. The tablets can also consist of a plurality of layers.

It is likewise possible to use the pharmaceutical composition of the invention for diagnosing infertility. This entails the amounts of HipK4 being determined for example by an ELISA assay or a protein chip.

The present invention also relates to the use of the pharmaceutical composition of the invention for diagnosing autoimmune antibodies in body fluids.

The present invention may additionally be employed as means for gene therapy through use of the pharmaceutical composition. This entails the effects of HipK4 being blocked through employing the gene therapy. In this connection, a vector which comprises a HipK4 antisense sequence is constructed and administered. Examples are vectors derived from adenovirus, adenovirus-associated virus, herpes simplex virus or SV40. Gene therapy can be carried out as described (Gomez-Navarro et al. 1999, Eur. J. Cancer, 35, 867-885). Administration can take place locally, i.e. directly into the abdominal cavity, or systemically, i.e. via the bloodstream. This leads to blockade of the expression of HipK4 in the testis. The biological function mediated by HipK4 is thus inhibited.

### Biological Examples:

### 1. Cloning and expression of human HipK4

The base pair 286-2136 segment of Seq ID No. 1 is amplified by standard PCR and cloned via the HindIII-NotI restriction cleavage sites into the expression vector pBB4.5. Baculoviruses for expression of HipK4 in insect cells are generated with the aid of the "Bac-N-Blue Kit" from Invitrogen in accordance with the manufacturer's instructions. Sf9 insect cells (11) are infected with an MOI (multiplicity of infection) of 3 with the HipK4 baculoviruses and cultured for 3 days. The cell pellet is then disrupted with a lysis buffer (50 mM Tris, pH 7.5, 150 mM NaCl, Protease Inhibitor Cocktail (Sigma)) and the HipK4 protein is purified using Anti-FLAG affinity columns (Sigma) in accordance with the instructions for use. The efficiency and purity of the purification is checked by a standard SDS gel electrophoresis with subsequent Western blotting (Fig. 3).

### 2. RNA preparation and tissue samples

For RNA preparation, the respective organs are transferred into liquid nitrogen and stored until processed further (human RNA samples were obtained from Contech). RNA is isolated using Trizol (Invitrogen), and the SuperScript III First Strand kit (Invitrogen) is used to synthesize the cDNA. Both methods are carried out strictly in accordance with the manufacturer's instructions. Testis and epididymis from wild type and HipK4-deficient mice are prepared for histological analysis and agitated in Bouin's fixative solution (0.9% picric acid, 9.5% formaldehyde and 4.8% acetic acid) overnight. The following day, the specimens are dehydrated in an increasing ethanol series and embedded in paraffin. The subsequently prepared 5 µM tissue sections can then be treated with a standard H&E stain.

### 3. Tissue distribution in human and murine tissue (TaqMan qPCR)

In order to detect the different levels of expression of HipK4 in the various human and mouse tissues, parts of the transcripts are quantified by PCR (Real-Time TaqMan, Applied Biosystems). The housekeeping gene *elongation factor 1 alpha (EF1α)* is used in order to normalize the amounts of cDNA. The PCR reactions are set up using the iQ-SYBR Green Supermix (BIO-RAD) in accordance with the standard protocol (manufacturer's instructions), using the following primer pairs (mouse HipK4 5'-CCGTCTGTCATCCCACAAC-3' and 5'-TAGGGTCCATTCTGGCTCAC-3'; human HipK4 5'-CCCGCTGCCCCTTCA-3' and 5'-GCACCTCGCTGAAAATGCT-3'). In order to reduce differences through pipetting errors, the reaction mixtures are combined by using a master mix which comprises the primers and the SYBR Green mix. The latter is then added to the cDNA. PCR programme: denaturation 95°C, 2 min; amplification (40 cycles) 94°C, 15 seconds and 60°C, 60 seconds. The DNA concentration is detected during the amplification. In order to reduce differences in expression between different individuals, always a plurality of tissue samples is combined to give a total pool. In order to reduce pipetting and PCR procedural errors, all PCR reactions are set up at least in triplicates. The average, taking account of the standard deviation, is used for the later quantification.

### 4. Tissue distribution in rat tissue by means of in-situ hybridization

Unless indicated otherwise, the incubation and washing steps are carried out at room temperature. The paraffin sections are dewaxed by immersion in xylene twice for 10 min each time and transferred into pure PBS by a decreasing ethanol/PBS series. This is followed by counterfixation in 4% PFA for 20 min, followed by washing in PBS twice for 5 min each time. After the sections have been subjected to a Proteinase K treatment for 10 min (10 mg/ml Proteinase K in 100 mM Tris-HCl, pH 7.5), they are immersed in 0.2% glycerol/PBS for 10 min to inhibit the reaction. Washing in PBS twice for 5 min is followed by fixation in 4% PFA for the second time for 10 min. The sections are then washed with PBS for 5 min, immersed in a 0.2 N HC1 solution for 15 min, again washed twice with PBS for 5 min and incubated in TEA solution (0.1 M triethanolamine, pH 8.0; 0.25% acetic anhydride) for 10 min in order to saturate amino groups. The sections are then washed in PBS and DEPC-H₂O for 5 min each in order to be subsequently prehybridized at 70°C for 2 h (in 50% formamide; 5 × SSC (20 × SSC: 3 M NaCl; 0.3 M Na citrate, pH 7.0), 50 µg/ml yeast tRNA; 1% SDS; 50 µg/ml heparin; 1 × Denhard's; 0.1% Tween). Hybridization takes place at 70°C overnight with 3 mg/ml digoxygenin-labelled sample in the prehybridization solution. On the following day, the sections are washed three times for 30 min in solution 1 (50% formamide; 5 × SSC pH 4.5 and 1% SDS in ddH₂O) at 67°C. Three incubations in TNT (10 mM Tris-HCl, pH 7.5; 0.5 M NaCl; 0.1% Tween-20) at RT for 5 min are followed by treatment of the sections with 100 µg/ml RNase in TNT at 37°C twice for 30 min. The sections are then, after a brief washing step in TNT:solution II (1:1) at RT for 5 min, incubated three times for 30 min in solution II (50% formamide; 2 × SSC pH 4.5; 0.2% SDS, in non-DEPC-treated water) at 63°C. After the sections are washed three times for 5 min each time in MAP solution (100 mM maleic acid; 150 mM NaCl; 2 mM levamisole, pH 7.5), blocking of the free binding sites in MAP/block/sheep serum (2% Roche blocking reagent in 100 mM maleic acid; 150 mM NaCl; 2 mM levamisole, pH 7.5; 10% sheep serum) followed at RT with constant agitation on a shaker for 3 hours. Finally, 200 µl of antibody solution (anti-digoxygenin) are applied to each slide and incubated at 4°C overnight. On the third day of ISH, the sections are washed in MAP solution for 10 min three times and for 1 hour four times. The sections are then rinsed with NTMT (100 mM Tris-HCl, pH 9.5; 50 mM MgCl2; 100 mM NaCl) three times for 10 min. The colour reaction is then carried out in BM Purple (Roche, Switzerland), 2 mM levamisole until the signal is identifiable, maximally overnight. The colour development is stopped by washing three times in PBT, pH 4.5, at RT for 5 min each time. The histological specimens are coverslipped in MOVIOL® for storage.

### 5. Activity detection (kinase assay)

Purified Hip kinase 4 is incubated in differing quantity together with 20 mM MOPS, pH 7.2, 25 mM β-glycerolphosphates, 5 mM EGTA, 1 mM Na₃VO₄, 1 mM dithiothreitol, 10 mM MgOAc, and 25 µM ATP (inc. 10 µCi of [γ-³³P]ATP) together with 25 µg of MBP (Upstate Biotechnology) in a final volume of 60 µl. After incubation at room temperature for two hours, 10 µl of the reaction mixture are put onto a Whatman P81 phosphocellulose paper. The filter is then washed with 0.75% phosphoric acid and dried, and the remaining radioactivity is measured. The result is depicted in Fig. 3. To identify putative substrates in an HTS method, the reaction volume is reduced to 15 µl, and the reaction is carried out in a 384-well microtitre plate. The peptides are diluted in water and added to the reaction mixture in a final concentration of 2.5 µM. The reaction is incubated with 10 ng of HipK4 protein for one hour and stopped by adding 50 mM EDTA, 3.33 mg/ml streptavidin-SPA beads (Amersham) in PBS. The plate is mixed for 10 min and centrifuged at 1000 rpm, and the scintillation signal is quantified using a Wallac microbeta reader.

### 6. Generation of a HipK4 knockout

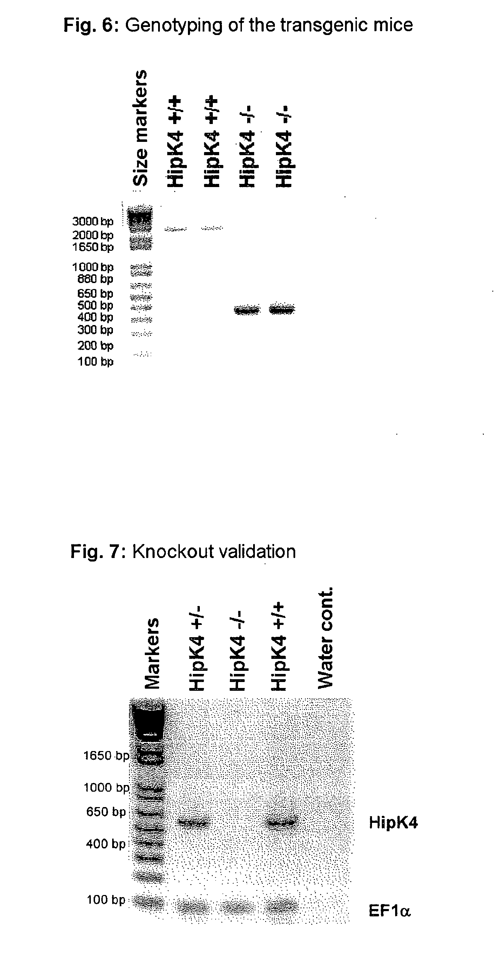

The targeting vector is cloned using a C57BL/6J BAC DNA collection (Artemis). Successful homologous recombination leads to deletion of exon 2-3 of the murine HipK4 allele and thus also to partial deletion of the protein kinase domain. The ES cell clone B-B2 (genetic background C57BL/6 N) is identified by Southern blotting as positive in relation to the homologous recombination (Fig. 5) (Artemis). For this purpose, the genomic DNA of various ES cell clones is digested with the restriction enzyme HindIII and fractionated on an agarose gel. After the digested DNA has been transferred by Southern blotting to a nitrocellulose membrane, the restriction fragment length polymorphisms (RFLPs) resulting from the homologous recombination can be detected by using a 3' external probe (Fig. 5). Two fragments are to be expected for wild-type clones, and 3 fragments for clones with successful homologous recombination on an allele: Fragments 11.2 kb and 8.7 kb in size for the wild-type clone, and an addition fragment 5.3 kb in size for clones with successful homologous recombination (HR). The ES cell clone B-B-2 is used for injection into C57BL/6 N blastocytes, and the chimeras resulting therefrom are mated with C57BL/6 N females. Genotyping of the transgenic offspring is carried out by PCR analysis employing genomic DNA from the tip of the tail. The primers employed for this, HipK4-A 5' CTACTGCACAACTAAATCTGTAGC-3' and HipK4-B 5'-CAGCGGTAGAGGAAGATAGAGG-3', hybridize in intron 2 (HipK4-A) and in intron 3 (HipK4-B) of the murine HipK4 gene. On use of these primers there is amplification of a fragment 364 base pair in size for the wild-type allele and 2850 base pair in size for the knockout allele (Fig. 6). Homozygous knockouts are generated by mating heterozygous mice. The absence of a HipK4 transcript in the homozygous knockout animals can be detected using a classical RT-PCR (Fig. 7).
The following primers are employed for this: HipK4-C 5'-TGCAGACTCAGGTCATCGAG-3' and HipK4-D 5'-GCAAGGCTCACCACTTCTTC-3' and the following primers for the control reaction with the *elongation factor 1 alpha:* E-A 5'AATTCACCAACACCAGCAGCAA-3' E-B 5'-TGCCCCAGGACACAGAGACTTCA-3').

### Figures:

Fig. 1: Expression in various tissues (TaqMan qPCR), human (Fig. 1B)/mouse (Fig. 1A) comparison.
   HipK4 is expressed principally in testes in mice and in humans (Fig. 1A and B). Expression in the brain was also detectable in mice, having 37% of the intensity of the testis expression level (Fig. 1B).
Fig. 2: Expression of HipK4 in rat testis (in situ hybridization)
   HipK4 is expressed exclusively in post-meiotic round spermatids. In combination with a PNA-lectin and propidium iodide staining, it was possible to restrict expression of HipK4 during spermiogenesis to development stage 4-6.
Fig. 3: Expression and purification of HipK4
   Standard SDS gel electrophoresis to check the efficiency and purity of the purification of the human HipK4.
Fig. 4: HipK4 phosphorylation of MBP
   The general kinase substrate MBP is phosphorylated. The intensity of the phosphorylation takes place as a function of the amount of HipK4 employed.
Fig. 5: Southern blot to detect the targeting
   Positive identification of the ES cell clone B-B2 by Southern blotting in relation to homologous recombination.
Fig. 6: Genotyping of the transgenic mice
   Genomic DNA is taken from the tip of the tail of transgenic offspring for the genotyping and is analysed by PCR.
Fig. 7: Knockout validation
   Demonstration of the absence of the HipK4 transcript in homozygous knockout animals by means of RT-PCR.
Fig. 8 and Fig. 9: Comparison of testes of wild-type with knockout animals
   Histological analysis of testis and epididymis. The deficiency of HipK4 in the testis leads to an influence on spermiogenesis (untypical arrangement of developing spermatids, Fig. 8, arrow). The defective spermiogenesis leads to release of a reduced number of sperm in the testis (Fig. 9).
Table 1: Identity between HipK4 and other proteins
   HipK4 shares only 74% sequence identity in the ATP binding pocket with its closest homologues, HipK1 and HipK2.

## Claims

1. Use of a pharmaceutical composition which contains as active component at least one substance selected from the group of a HipK4 nucleic acid corresponding to Seq ID No. 1, a HipK4 polypeptide corresponding to Seq ID No. 2, modulators of HipK4, antibodies directed against HipK4, a HipK4 antisense, and a HipK4 RNAi, for the diagnosis and treatment of fertility impairments, for contraception and also for manufacturing a medicament for the treatment of fertility impairments.

2. Use of the pharmaceutical composition according to Claim 1, where the nucleic acid includes a protein-coding segment which corresponds to the nucleic acid sequence depicted in Seq ID No. 1.

3. Use of the pharmaceutical composition according to Claim 1, where the nucleic acid codes for a polypeptide having the amino acid sequence depicted in Seq ID No. 2.

4. Use according to Claim 3, where the protein-coding segment is located in the region of nucleotide 286-2136 of the sequence depicted in Seq ID No. 1.

5. Use of the pharmaceutical composition according to Claim 1, where the contained HipK4 polypeptide includes a polypeptide fragment which is located in the region from position 1 to position 616 of the amino acid sequence shown in Seq ID No. 2.

6. Use of the pharmaceutical composition according to Claim 1, where the contained antibodies are directed against the HipK4 polypeptide, for diagnosing fertility impairments.

7. Use of the pharmaceutical composition according to Claim 1, where the composition comprises a nucleic acid probe which is complementary to the nucleic acid sequences which codes for the polypeptide which includes the amino acid sequence depicted in Seq ID No. 4, for diagnosing fertility impairments.

8. Use according to Claim 7, where the nucleotide sequences are provided with a detectable marker.

9. Use of the pharmaceutical composition according to Claim 1, where the composition comprises molecules which are able to suppress the expression of HipK4, for contraception.

10. Use according to Claim 9, where the molecule is an antisense molecule.

11. Use of the pharmaceutical composition according to Claim 1, which comprises as active component at least one HipK4 nucleic acid, or a HipK4 polypeptide or a HipK4 antisense molecule as target substance for producing a composition for the treatment of diseases which are causally related to the HipK4 gene and/or protein.

12. Use of the pharmaceutical composition according to Claim 1 as means for gene therapy, where the active component is a HipK4 nucleic acid.

13. Method for producing the proteins or polypeptides which are present in the pharmaceutical composition according to Claim 4 and 5, **characterized in that** host cells are cultured under conditions which permit expression of the DNA sequence, and whereby the expression product can be obtained from the culture mixture.

14. Use of the proteins or polypeptides which are present in the pharmaceutical composition according to Claim 1 for isolating and preparing specific ligands.

15. Use of a pharmaceutical composition according to Claim 1, which comprises a HipK4 nucleic acid, a HipK4 polypeptide or a cell which expresses HipK4, for identifying effectors of a HipK4 polypeptide.

16. Assay system for identifying effectors of a HipK4 polypeptide, where a HipK4 polypeptide as a whole or partial sequences thereof is incubated with a modulator, and the binding of a molecule to the HipK4 polypeptide is measured, where HipK4 is incubated with radiolabelled ATP and the substrate MBP and, after an appropriate incubation time, the degree of phosphorylation of the substrate is determined.

17. Use of the pharmaceutical composition according to Claim 1, where the effector is an inhibitor of HipK4, for contraception.

18. Method for providing a pharmaceutical composition, where
a) substances are contacted with an assay system for identifying HipK4 effectors,
b) the effect of the substances on the assay system is measured by comparison with controls,
c) a substance which shows a modulation of the activity of the HipK4 polypeptide in step b) is identified,
d) and the substance identified in step c) is mixed with formulating materials customary in pharmacy.

19. Use of a DNA chip of the invention for diagnosing fertility impairments preferably in the testis and oviduct.

20. Use of the pharmaceutical composition according to Claim 1, which comprises an siRNA which results in modification of the HipK4 transcript, for contraception.
